# EUROPÄISCHE PATENTSCHRIFT

(11) **EP 0 340 866 B1**
(45) Veröffentlichungstag und Bekanntmachung des Hinweises auf die Patenterteilung: **17.08.1994**
(21) Anmeldenummer: 89201116.4
(22) Anmeldetag: 01.05.1989
(51) Int. Cl.: H04N 5/32

(54) **Anordnung zur Erzeugung von Röntgenaufnahmen mittels eines Photoleiters**
Device for producing x-rays pictures using photoconductors
Dispositif de production d'images radiologiques au moyen de photoconducteurs

(30) Priorität: 06.05.1988 DE 3815458
(43) Veröffentlichungstag der Anmeldung: 08.11.1989
(73) Patentinhaber: Philips Patentverwaltung GmbH, 22335 Hamburg (DE); Philips Electronics N.V., 5621 BA Eindhoven (NL)
(72) Erfinder: Hillen, Walter, Dr., D-5100 Aachen (DE); Quadflieg, Peter, D-5100 Aachen (DE); Schiebel, Ulrich, Dr., D-5100 Aachen (DE)
(74) Vertreter: Hartmann, Heinrich, Dipl.-Ing.

(56) Entgegenhaltungen:
- EP-A- 0 131 982
- EP-A- 0 154 042
- GB-A- 1 496 748
- US-A- 4 136 942

## Beschreibung

Die Erfindung betrifft eine Anordnung zur Erzeugung von Röntgenaufnahmen mittels eines Röntgenstrahlung in ein Ladungsmuster umsetzenden Photoleiters, der vor der Röntgenaufnahme aufgeladen wird und dessen Oberfläche nach der Aufnahme zur Erfassung des Ladungsmusters abgetastet wird. Eine entsprechende Anordnung ist im wesentlichen aus der DE-A-29 48 660 bekannt.

Ein idealer Photoleiter ist ein Isolator, wenn er nicht belichtet wird. Lediglich während einer Belichtung bzw. einer Bestrahlung mit Röntgenstrahlen wird er leitfähig, und zwar umso mehr, je höher die Bestrahlungsintensität ist. Damit wird an den bestrahlten Stellen die durch eine vorherige Aufladung erzeugte Ladungsdichte entsprechend der dort auftreffenden Dosis verringert. Das auf diese Weise auf der Oberfläche des Photoleiters erzeugte zweidimensionale Ladungsmuster, das im wesentlichen der räumlichen Verteilung der Röntgenstrahlungsdosis entspricht ("latentes Bild" oder "Ladungsbild") wird von der Abtasteinrichtung in elektrische Signale umgesetzt. Diese werden verstärkt gefiltert digitalisiert und abgespeichert. Sie sind dann der digitalen Bildverarbeitung zugänglich.

Bei der Herstellung von Röntgenaufnahmen mit einer solchen Anordnung können sich in der auf diese Weise erzeugten Röntgenaufnahme Artefakte ergeben, wenn in geringem zeitlichen Abstand eine andere Röntgenaufnahme vorausgegangen ist. Aufgabe der vorliegenden Erfindung ist es, eine Anordnung der eingangs genannten Art so auszugestalten, daß das Ausmaß der geschilderten Artefakte verringert wird. Diese Aufgabe wird erfindungsgemäß dadurch gelöst, daß eine Nachladevorrichtung vorgesehen ist, die die Abnahme der Ladungsdichte an der Oberfläche des Photoleiters während der Röntgenaufnahme auf einen vorgegebenen Wert begrenzt.

Die Erfindung basiert auf der Erkenntnis, daß die geschilderten Artefakte darauf beruhen, daß sich nach einer Wiederaufladung des Photoleiters im Anschluß an eine Röntgenaufnahme diejenigen Stellen, die bei der vorangegangenen Aufnahme besonders stark von Röntgenstrahlung getroffen worden sind, besonders schnell entladen, und zwar auch im Dunkeln, und daß dieser Effekt dadurch bedingt ist, daß der Photoleiter an den betreffenden Stellen während der Röntgenaufnahme besonders stark entladen war. Dadurch, daß die Entladung während der Aufnahme erfindungsgemäß auf einen vorgegebenen Wert begrenzt wird, wird eine solche starke Entladung verhindert und damit auch, daß sich der Photoleiter nach seiner Wiederaufladung an den betreffenden Stellen besonders schnell entlädt.

Die Nachladevorrichtung besteht im Prinzip aus einer Ladungsträgerquelle, deren Potential so gewählt ist, daß die Oberfläche des Photoleiters aufgeladen wird, wenn die Ladungsdichte einen vorgebbaren Wert unterschreitet. Eine bevorzugte Ausgestaltung einer derartigen Nachladevorrichtung ist dadurch gekennzeichnet, daß die Nachladevorrichtung eine Koronaentladungs-Vorrichtung enthält, daß zwischen dieser und dem Photoleiter ein Gitter angeordnet ist, das den bei einer Röntgenaufnahme belichtbaren Bereich des Photoleiters überdeckt und das das Potential der Photoleiteroberfläche auf einen annähernd dem Gitterpotential entsprechenden Wert begrenzt.

In weiterer Ausgestaltung der Erfindung ist vorgesehen, daß die Koronaentladungs-Vorrichtung zur Aufladung des Photoleiters vor einer Röntgenaufnahme dient und daß die Spannung zwischen dem Gitter und dem leitenden Substrat, auf dem der Photoleiter angeordnet ist, nach dieser Aufladung auf einen niedrigeren Wert herabsetzbar ist.

Eine Koronaentladungs-Vorrichtung besitzt einen oder mehrere parallele Drähte, die im Betriebszustand an eine hohe Spannung gelegt werden, so daß sich eine Koronaentladung ergibt. Um zu vermeiden, daß diese Drähte auf der Röntgenaufnahme abgebildet werden, ist nach einer Weiterbildung der Erfindung vorgesehen, daß eine Antriebseinrichtung zur Erzeugung einer Relativbewegung zwischen dem Gitter und der Oberfläche des Photoleiters vorgesehen ist.

Die Erfindung wird nachstehend anhand der Zeichnung näher erläutert. Es zeigen:
Fig. 1 den zeitlichen Verlauf der Potentialänderungsgeschwindigkeit auf der Oberfläche eines Photoleiters bei unterschiedlichen Belichtungen,
Fig. 2 eine Anordnung nach der Erfindung,
Fig. 3 den Verlauf des Oberflächenpotentials ohne und mit der erfindungsgemäßen Maßnahmen und
Fig. 4 eine weitere Ausführungsform der Erfindung.

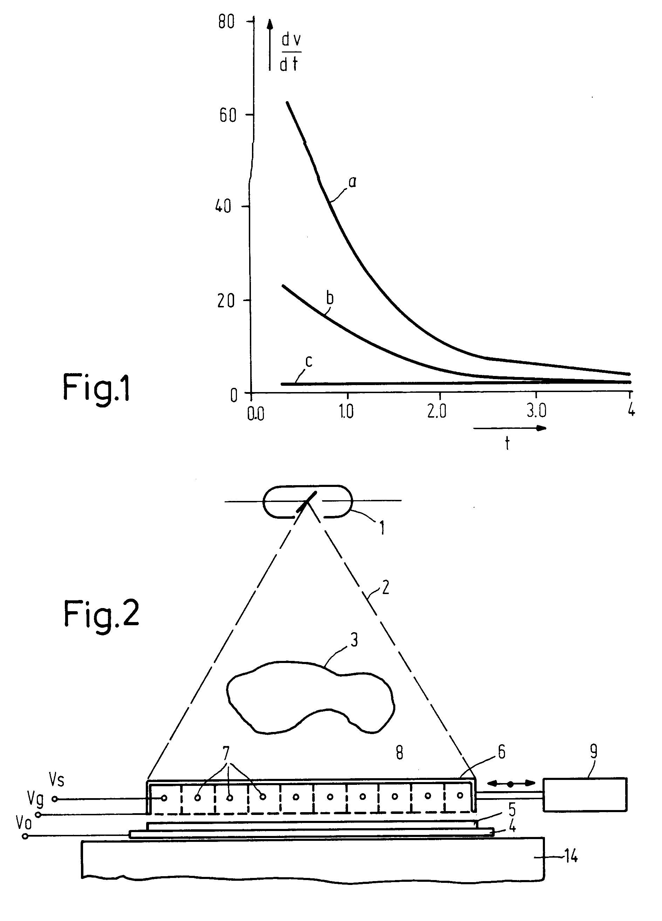

Wird die Oberfläche eines Photoleiters, beispielsweise aus amorphem Selen mit einer relativ hohen Strahlendosis ( 100 µGy) bestrahlt, so ist nach sofort anschließender Aufladung die Dunkelentladungsrate, d.h. die Änderung des Oberflächenpotentials pro Zeiteinheit bei unbestrahltem Photoleiter zunächst stark erhöht. Im Laufe weniger Minuten reduziert sie sich dann wieder auf ihren normalen Wert. In Fig. 1 zeigt die Kurve a einen typischen zeitlichen Verlauf der Dunkelentladungsrate (ausgedrückt in V/min) im Anschluß an eine Röntgenbelichtung mit einer Dosis von 1 mGy; die Zeit ist dabei in Minuten angegeben. Zum Vergleich ist mit Kurve c die Dunkelentladungsrate des gleichen Photoleiters ohne vorherige Belichtung dargestellt.

Eine Dosis in der Größenordnung von 1 mGy wird bei Anwendungen im Bereich der medizinischen Röntgendiagnostik häufig zumindest auf Teile des Photoleiters treffen, weil die durchstrahlten Objekte zum Teil einen sehr großen Anteil der Strahlung absorbieren. Im eigentlichen Bildbereich treffen dann zwar nur Dosen der Größenordnung 1 bis 10 µGy auf den Photoleiter, aber es ist unvermeidlich, daß gewisse Bereiche des Photoleiters der ungeschwächten Direktstrahlung ausgesetzt werden.

Bei einer Aufnahmefrequenz von 1/min oder mehr ergibt sich gemäß Fig. 1 die Situation, daß sich die Dunkelentladungsrate in den bei der voraufgegangenen Aufnahme durch Direktstrahlungseinwirkung stark vorbelasteten Bereichen des Photoleiters zeitlich stark verändert. Infolgedessen ist zu dem Zeitpunkt, zu dem das Ladungsmuster auf der Oberfläche des Photoleiters mit einer Influenzsondenanordnung abgetastet wird, das Ladungsmuster nicht mehr die exakte Wiedergabe des Röntgenstrahlenbildes, was die eingangs geschilderten Artefacte zur Folge hat. Damit ist auch eine Korrektur des Dunkelentladungseffektes durch die Subtraktion eines Dunkelentladungsbildes, wie sie in der deutschen Patentanmeldung P 35 29 108 beschrieben ist, erheblich erschwert.

Die geschilderte erhöhte Dunkelentladungsrate wird durch die Erfindung wesentlich verringert.

Eine Anordnung nach der Erfindung ist in Fig. 2 dargestellt. Mit 1 ist dabei ein Röntgenstrahler bezeichnet, der ein Röntgenstrahlenbündel 2 erzeugt, das einen Patienten 3 durchsetzt und das von einer Photoleiteranordnung in ein Ladungsmuster umgesetzt wird. Die Photoleiteranordnung besteht aus einer auf einer Aluminiumelektrode 4 aufgebrachten 0,5 mm starken Schicht 5 aus Selen, die mit 0,5 % Arsen dotiert ist. Die Aluminiumelektrode ist auf ein geeignetes isolierendes Substrat 14 aufgebracht. In einem geringen Abstand von der Selenschicht 5 befindet sich eine gittergesteuerte Koronaentladungs-Vorrichtung. Diese umfaßt ein Gehäuse 6, dessen Material und Wandstärke so gewählt sind, daß die Röntgenstrahlung dadurch nur unwesentlich geschwächt wird. Es kann aus einem dünnen Aluminiumblech oder aus Kunststoff bestehen, der auf einer Seite mit einer dünnen Schicht metallisiert ist. Die leitende Gehäusewand ist geerdet. Die Gehäuseabmessungen entsprechen den Abmessungen der Photoleiterschicht.

In dem Gehäuse befinden sich parallel zueinander und zur Oberfläche des Photoleiters 5 angeordnete Drähte 7, die elektrisch leitend miteinander verbunden sind. Auf seiner dem Photoleiter zugewandten Seite ist das Gehäuse durch ein elektrisch steuerbares Gitter 8 abgeschlossen. Das Gitter hat einen geringen Abstand zum Photoleiter (typisch 0,2 bis 2,0 mm) und eine geringe Maschenweite (typisch 0,1 bis 0,5 mm).

Vor der Röntgenaufnahme wird die Oberfläche des Photoleiters 5 aufgeladen. Dazu wird die Aluminiumelektrode 4 an eine Spannung Vo von z.B. -1.750 V gelegt (gegenüber dem Gehäuse 6). An die Drähte 7 wird eine positive Hochspannung Vs angelegt, deren Betrag so gewählt wird, daß die Oberfläche des Photoleiters innerhalb einer Zeit von 10 bis 100 ms auf das Potential des Gitters Vg aufgeladen wird. Das Gitterpotential entspricht entweder Erdpotential (0 V) oder ist leicht negativ, so daß alle durch die Koronaentladung um die Drähte 7 herum erzeugten Ladungsträger zum Gitter hin beschleunigt werden. Wenn das Potential an der Oberfläche des Photoleiters das Gitterpotential erreicht hat, werden die von den Drähten 7 emittierten Ladungsträger vom Gitter abgefangen und gelangen nicht mehr zum Photoleiter. Auf dessen Oberfläche stellt sich daher relativ genau das gewünschte Oberflächenpotential ein, wobei die durch den Aufbau der gittergesteuerten Koronaentladungsvorrichtung 6 .. 8 bedingten Schwankungen des Oberflächenpotentials kleiner sind als 0,1 V.

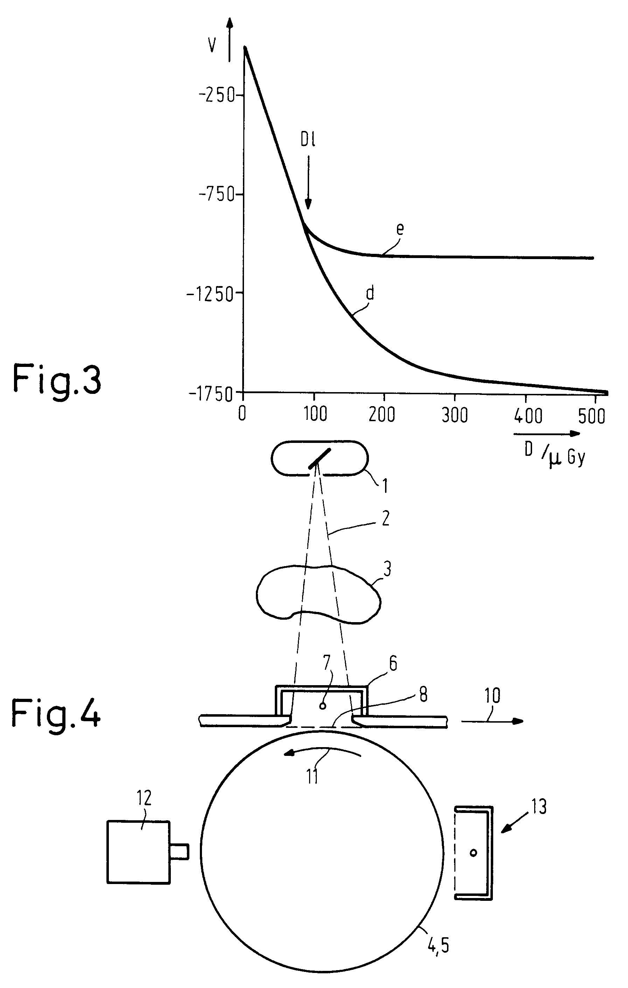

Wenn nach der Aufladung bzw. bei Beginn der Röntgenaufnahme die Koronaentladung abgeschaltet würde, dann würde sich der Photoleiter 5 bei der nachfolgenden Röntgenaufnahme mit einer Rate entladen, die im wesentlichen proportional zur Röntgendosisleistung ist. Die Entladung stoppt erst, wenn die Ladung an der Oberfläche des Photoleiters abgebaut ist, es sei denn, die Röntgenröhre 1 würde vorher abgeschaltet. Dabei würde das Oberflächenpotential als Funktion der bei der Röntgenaufnahme erhaltenen Röntgendosis (gemessen in µGy) den in Fig. 3 durch die Kurve d dargestellten typischen Verlauf aufweisen.

Erfindungsgemäß wird bei Röntgenaufnahme die Koronaentladungsvorrichtung jedoch nicht abgeschaltet, sondern weiterbetrieben, und es wird lediglich die Differenz Vg-Vo zwischen Gitter- und Substratpotential verringert, beispielsweise dadurch, daß das Steuergitter 8 mit einem negativen Potential Vg von -900 V betrieben wird. Dadurch wird die Entladung des Photoleiters gestoppt, sobald sein Oberflächenpotential an irgendeiner Stelle den Wert des Gitterpotentials erreicht hat. Das Steuergitter wird dann nämlich für positive Ladungsträger durchlässig, so daß diese auf die entsprechenden Stellen der Oberfläche des Photoleiters gelangen und den durch die Bestrahlung induzierten Entladungsstrom kompensieren. Das Oberflächenpotential als Funktion der Bestrahlungsdosis hat dann den in Fig. 3 durch die Kurve e gekennzeichneten Verlauf, der bis zu einer Grenzdosis Dl von etwa 100 µGy mit dem der Kurve d identisch ist, danach aber von der Bestrahlungsdosis weitgehend unabhängig ist.

Die Kurve b in Fig. 1 zeigt den zeitlichen Verlauf der Dunkelentladungsrate bei einem Photoleiter, der entsprechend der Kurve e in Fig. 3 bei Unterschreiten eines Grenzwertes der Ladungsdichte bzw. des Potentials an der Oberfläche nachgeladen wird, so daß sich an den betreffenden Stellen ein vorgegebenes Potential bzw. eine vorgegebene Ladungsdichte einstellt. Man erkennt, daß durch diese Begrenzung der Ladungsdichte bzw. des Potentials an der Oberfläche der Dunkelentladungseffekt im Vergleich zu einer Dunkelentladung ohne eine Nachladung bei der vorangehenden Aufnahme (Kurve a) deutlich herabgesetzt ist.

Durch die Begrenzung der Ladungsdichte bzw. des Oberflächenpotentials wird allerdings auch der Dynamikbereich des Photoleiters eingeschränkt, und die in den nachgeladenen Bereichen enthaltene Bildinformation geht verloren. Im Beispiel der Kurve e in Fig. 3 liegt die Grenzdosis bei ungefähr 100 µGy, sie kann bei Bedarf durch Erhöhung der Elektrodenspannung Vo oder durch Erniedrigung von Vg bis auf etwa 200 µGy erhöht werden. In der medizinisch diagnostischen Anwendung liegen die mittleren Dosen zwischen 1 und 10 µGy. Damit verbleibt im allgemeinen ein genügend großer Belichtungsspielraum nach oben.

Bei der in der Projektionsradiographie üblichen Belichtungstechnik ist der Photoleiter eben und hat maximale Abmessungen von 450 x 450 mm² Quadrat; alle Bereiche des Photoleiters werden gleichzeitig belichtet. Zur Durchführung des Nachladeverfahrens ist daher eine Koronaeinrichtung erforderlich, die die gesamte Sektorfläche abdeckt. Die Drähte 7 und das Gitter 8 können bei einer Röntgenaufnahme abgebildet werden, da sie Röntgenstrahlung absorbieren. Diese Abbildung kann jedoch verhindert werden, indem das Gehäuse 6 mitsamt den darin befindlichen Komponenten durch eine Antriebseinrichtung 9 während der Röntgenaufnahme mit geeigneter Frequenz und Amplitude hin- und herbewegt wird (ähnlich wie bei einem Streustrahlenraster), so daß die Strukturen dieser Teile im Bild verwischt werden.

Wesentlich einfacher ist die Gestaltung der Koronaeinrichtung, wenn das Verfahren in einem Gerät für Schlitzradiographie eingesetzt wird, wie es z.B. aus der DE-OS 35 34 768 bekannt ist. Ein solches Gerät ist in Fig. 4 dargestellt und wird im folgenden kurz erläutert. Von der von dem Röntgenstrahler 1 emittierten Röntgenstrahlung wird nur ein vergleichsweise schmales Strahlenbündel 2 ausgeblendet, das nur einen Teil des Untersuchungsobjektes durchsetzt und nur einen Teil des Photoleiters 4, 5 belichtet, der sich auf der Oberfläche einer sich während der Röntgenaufnahme drehenden Trommel befindet. Die Abbildung erfolgt dadurch, daß die Oberfläche des Photoleiters 4, 5 sowie das Objekt 3 einerseits und das Strahlenbündel 2 andererseits relativ zueinander bewegt werden, wie durch die Pfeile 10 und 11 angedeutet, so daß auf der zylindrischen Oberfläche des Photoleiters 4, 5 das Röntgenstrahlenbild des gesamten Aufnahmeobjektes 3 erscheint, das mittels einer schematisch dargestellten Influenzsondenanordnung 12 abgetastet und digital weiterverarbeitet werden kann.

Die Koronaentladungs-Vorrichtung muß dabei lediglich den Schlitz der Photoleiteroberfläche überdecken, der bei der Röntgenaufnahme jeweils belichtet wird. Die Abmessungen der Vorrichtung 6..8 können daher wesentlich reduziert werden. Die Relativbewegung zwischen Photoleiter und Belichtungsschlitz verhindert, daß die Strukturen der Koronaentladungs-Vorrichtung auf der Röntgenaufnahme abgebildet werden. Eine zusätzliche Bewegungseinrichtung (wie die die Einrichtung 9 in Fig. 2) ist daher nicht nötig. Die für die Nachladung bestimmte Koronaentladungsvorrichtung 6, 7, 8 kann ebenso wie bei der Anordnung nach Fig. 2 zur Aufladung des Photoleiters 4, 5 vor der Röntgenaufnahme dienen. In diesem Fall kann die der Aufladung dienende, außerhalb des Strahlenganges befindliche Koronaentladungs-Vorrichtung 13 entfallen.

## Patentansprüche

1. Anordnung zur Erzeugung von Röntgenaufnahmen mittels eines Röntgenstrahlung in ein Ladungsmuster umsetzenden Photoleiters, der vor der Röntgenaufnahme aufgeladen wird und dessen Oberfläche nach der Aufnahme zur Erfassung des Ladungsmusters abgetastet wird,
dadurch gekennzeichnet, daß eine Nachladevorrichtung (6..9) vorgesehen ist, die die Abnahme der Ladungsdichte an der Oberfläche des Photoleiters (5)während der Röntgenaufnahme auf einen vorgegebenen Wert begrenzt.

2. Anordnung nach Anspruch 1,
dadurch gekennzeichnet, daß die Nachladevorrichtung eine Koronaentladungs-Vorrichtung (6,7) enthält, daß zwischen dieser und dem Photoleiter ein Gitter (8) angeordnet ist, das den bei einer Röntgenaufnahme belichtbaren Bereich des Photoleiters überdeckt und das das Potential der Photoleiteroberfläche auf einen annähernd dem Gitterpotential entsprechenden Wert begrenzt.

3. Anordnung nach Anspruch 2,
dadurch gekennzeichnet, daß die Koronaentladungs-Vorrichtung zusätzlich zur Aufladung des Photoleiters vor einer Röntgenaufnahme dient und daß die Spannung zwischen dem Gitter und dem leitenden Substrat, auf dem der Photoleiter angeordnet ist, nach dieser Aufladung auf einen niedrigeren Wert herabsetzbar ist.

4. Anordnung nach Anspruch 2,
dadurch gekennzeichnet, daß eine Antriebseinrichtung (9) zur Erzeugung einer Relativbewegung zwischen der Koronaentladungs-Vorrichtung und der Oberfläche des Photoleiters vorgesehen ist.

## Claims

1. A device for producing X-ray images by means of a photoconductor which converts X-rays into a charge pattern, which is charged prior to the X-ray exposure, and whose surface is scanned after exposure in order to detect the charge pattern, characterized in that there is provided a recharging device (6 ... 9) which limits the decrease of the charge density on the surface of the photoconductor (5) during the X-ray exposure to a predetermined value.

2. A device as claimed in Claim 1, characterized in that the recharging device comprises a corona discharge device (6,7), between said corona discharge device and the photoconductor there being arranged a grid (8) which covers the area of the photoconductor which can be exposed during an X-ray exposure and which limits the potential of the photoconductor surface to a value which corresponds approximately to the grid potential.

3. A device as claimed in Claim 2, characterized in that the corona discharge device also serves for charging the photoconductor prior to a X-ray exposure, it being possible to reduce the voltage between the grid and the conductive substrate supporting the photoconductor to a lower value after such charging.

4. A device as claimed in Claim 2, characterized in that there is provided a drive (9) for creating a relative motion between the corona discharge device and the surface of the photoconductor.

## Revendications

1. Dispositif de production d'images radiologiques au moyen d'un photoconducteur convertissant les rayons X en un motif de charges, qui est chargé avant la prise d'image et dont la surface est balayée après cette prise d'image en vue de la saisie du motif de charges, caractérisé en ce qu'un dispositif de recharge (6..9) est prévu, lequel dipsositif limite à une valeur donnée la diminution de la densité de charge à la surface du photoconducteur (5) pendant la prise d'image.

2. Dispositif selon la revendication 1, caractérisé en ce que le dispositif de recharge comprend un dispositif de décharge à effet de couronne (6, 7), une grille (8) qui est disposée entre ce dispositif et le photoconducteur et recouvre la zone du photoconducteur qui peut être exposée lors de la prise d'image et limite le potentiel de la surface du photoconducteur à une valeur correspondant approximativement au potentiel de la grille.

3. Dispositif selon la revendication 2, caractérisé en ce que le dispositif de décharge à effet de couronne sert, en outre, à charger le photoconducteur avant une prise d'image et en ce que la tension entre la grille et le substrat conducteur, sur lequel le photoconducteur est disposé, peut être abaissée à une valeur inférieure après cette charge.

4. Dispositif selon la revendication 2, caractérisé en ce qu'un mécanisme d'entraînement (9) est prévu pour produire un mouvement relatif entre le dispositif de décharge à effet de couronne et la surface du photoconducteur.
